(19) **Europäisches Patentamt / European Patent Office / Office européen des brevets**

(11) **EP 2 687 238 B1**

(12) **EUROPEAN PATENT SPECIFICATION**

(45) Date of publication and mention
of the grant of the patent:
**10.07.2019 Bulletin 2019/28**

(21) Application number: **12757389.7**

(22) Date of filing: **07.03.2012**

(51) Int Cl.:
*A61L 17/00* (2006.01)     *D02G 3/28* (2006.01)
*D02G 3/38* (2006.01)     *D02G 3/46* (2006.01)
*A61L 17/12* (2006.01)     *A61L 17/14* (2006.01)
*D02G 3/44* (2006.01)

(86) International application number:
**PCT/JP2012/055788**

(87) International publication number:
**WO 2012/124562 (20.09.2012 Gazette 2012/38)**

(54) **BIORESORBABLE SUTURE THREAD AND PRODUCTION METHOD FOR BIORESORBABLE SUTURE THREAD**

BIORESORBIERBARER NAHTFADEN UND HERSTELLUNGSVERFAHREN FÜR DEN BIORESORBIERBAREN NAHTFADEN

FIL DE SUTURE BIORÉSORBABLE ET PROCÉDÉ DE FABRICATION D'UN FIL DE SUTURE BIORÉSORBABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2011 JP 2011056579**

(43) Date of publication of application:
**22.01.2014 Bulletin 2014/04**

(73) Proprietor: **Gunze Limited**
**Ayabe-shi, Kyoto 623-8511 (JP)**

(72) Inventors:
• **KISHIDA, Yasuyuki**
 **Ayabe-shi**
 **Kyoto 623-8513 (JP)**
• **MAEHARA, Naoki**
 **Ayabe-shi**
 **Kyoto 623-8513 (JP)**

• **MORI, Yukio**
 **Ayabe-shi**
 **Kyoto 623-8513 (JP)**
• **WATANABE, Mariko**
 **Ayabe-shi**
 **Kyoto 623-8513 (JP)**

(74) Representative: **Adam, Holger et al**
 **Kraus & Weisert**
 **Patentanwälte PartGmbB**
 **Thomas-Wimmer-Ring 15**
 **80539 München (DE)**

(56) References cited:
**WO-A1-93/03213     WO-A1-03/092758**
**WO-A2-2006/058305     JP-A- 10 216 218**
**JP-A- 59 001 774     JP-A- H06 509 400**
**JP-A- 2001 079 079     US-A- 4 470 941**

**Description**

TECHNICAL FIELD

The present invention relates to a bioabsorbable suture thread that has high flexibility and high tensile and knot strengths, maintains its figure without untwisting a plurality of threads even at a cut end or a point of torsional bending, and is excellent in knot security and production efficiency, and a production method for the bioabsorbable suture thread.

BACKGROUND ART

Surgical suture threads are one of medical devices traditionally used. Biodegradable and bioabsorbable suture threads, which do not have to be removed after surgery, have been frequently used in recent years. Suture threads made of raw materials such as polyglycolic acid are commercially available as such absorbable suture threads.

Monofilament suture threads consisting only of single fibers and multifilament suture threads consisting of a plurality of fibers are known as forms of the surgical suture threads. Among them, highly flexible multifilament suture threads are suitable for, for example, use in the inosculation or suture of digestive organs with a lot of movements, such as the stomach or intestinal tracts. Braided suture threads composed of a plurality of multifilament threads weaved in a braided form using a braiding machine are known to be able to particularly establish both high flexibility and high tensile and knot strengths (e.g., Patent Literatures 1 to 6).

The braided suture thread, however, disadvantageously becomes separated due to its braid that comes loose at an end when the thread is cut. In the case of the inosculation or suture of complicated tissues, torsional bending may be applied to the suture thread. The conventional braided suture thread placed under such torsional bending also disadvantageously becomes separated at a point of torsional bending. A further problem of the conventional braided suture thread is lower knot security in such a way that its knot tends to come loose due to such separation of the thread. In the production of the braided suture thread, multifilament threads obtained by spinning are processed into a braided form using a braiding machine. Unfortunately, such a production method requires many production steps and is thus disadvantageous in terms of production efficiency or cost.

CITATION LIST

- Patent Literature

Patent Literature 1: Japanese Kokai Publication No. Hei-06-14987 (JP-A H06-14987)
Patent Literature 2: Japanese Kokai Publication No. 2008-113790 (JP-A 2008-113790)
Patent Literature 3: Japanese Kokai Publication No. 2004-250853 (JP-A 2004-250853)
Patent Literature 4: US 4,470,941 A
Patent Literature 5: WO 03/092758 A1
Patent Literature 6: WO 93/03213 A1

SUMMARY OF INVENTION

- Technical Problem

In light of the circumstances described above, an object of the present invention is to provide a bioabsorbable suture thread that has high flexibility and high tensile and knot strengths, maintains its figure without untwisting a plurality of threads even at a cut end or a point of torsional bending, and is excellent in knot security and production efficiency, and a production method for the bioabsorbable suture thread.

- Solution to Problem

The present invention provides the bioabsorbable suture thread according to claim 1.

As a result of diligent studies, the present inventors have completed the present invention by finding that a bioabsorbable suture thread that has high flexibility and high tensile and knot strengths, maintains its figure without untwisting a plurality of threads even at a cut end or a point of torsional bending, and is excellent in knot security and production efficiency can be obtained by: winding a plurality of winding threads made of a bioabsorbable higher-melting component around a core thread made of a bioabsorbable lower-melting component; and melting the core thread by heat treatment to bond the core thread to the winding threads.

The bioabsorbable suture thread of the present invention comprises, inter alia: a core thread made of a bioabsorbable lower-melting component; and a plurality of winding threads made of a bioabsorbable higher-melting component, the winding threads being wound around the core thread.

The core thread is molten by heat treatment so that the core thread plays a role as a bonding resin for bonding the core thread to the winding threads.

The bioabsorbable lower-melting component constituting the core thread has a melting point of 50 to 200°C. The lower-melting component having a melting point lower than 50°C may run off due to complete melting during production steps. Thus, the resulting core thread might be unable to exert its effect of bonding a plurality of winding threads. If the melting point of the lower-melting component exceeds 200°C, this melting point does not much differ from that of the bioabsorbable higher-melting component constituting each of a plurality of the winding threads wound therearound and might cause the core thread and a plurality of the winding threads wound therearound to be molten together during production. The melting point of the lower-melting component is preferably 60°C as the lower limit and 160°C as the upper limit.

In the present specification, the melting point means a value obtained by differential scanning calorimetry (DSC) measurement.

The bioabsorbable lower-melting component constituting the core thread is at least one selected from the group consisting of ε-polycaprolactone, a copolymer of ε-polycaprolactone and glycolic acid, and a copolymer of ε-polycaprolactone and

lactic acid, because a flexible core thread can be prepared.

The core thread may be a monofilament thread or a multifilament thread and can be arbitrarily selected in consideration of the number of the winding threads wound therearound, the total diameter of the suture thread, etc.

Preferably, the core thread has a diameter of 0.03 to 0.25 mm. If the diameter of the core thread is smaller than 0.03 mm, a plurality of the winding threads might be difficult to wind around the core thread. Alternatively, the core thread might be insufficiently bonded to the winding threads even after being molten by heat treatment. The core thread having a diameter exceeding 0.25 mm is too thick. Thus, the resulting suture thread might lose its flexibility. The diameter of the core thread is more preferably 0.05 mm as the lower limit and 0.2 mm as the upper limit.

The bioabsorbable suture thread of the present invention has a plurality of winding threads made of a bioabsorbable higher-melting component, the winding threads being wound around the core thread.

The bioabsorbable higher-melting component constituting the winding threads has a melting point of 180 to 290°C. If the melting point of the higher-melting component is lower than 180°C, this melting point does not much differ from that of the bioabsorbable lower-melting component constituting the core thread and might cause the core thread and a plurality of the winding threads wound therearound to be molten together during production. The higher-melting component having a melting point exceeding 290°C makes spinning by a usual spinning method difficult. The melting point of the higher-melting component is preferably 200°C as the lower limit and 270°C as the upper limit.

The bioabsorbable higher-melting component constituting the winding threads is polyglycolic acid and/or a copolymer of glycolic acid and lactic acid, because an absorbable suture thread having high tensile and knot strengths can be prepared.

The winding threads may be monofilament threads or multifilament threads and are preferably multifilament threads because a bioabsorbable suture thread having high flexibility can be prepared.

Multifilament threads each composed of 10 or more monofilament threads are preferred as the winding threads because such multifilament threads can achieve higher flexibility.

Preferably, the winding threads each have a diameter of 0.05 to 0.6 mm. If the respective diameters of the winding threads are smaller than 0.05 mm, the threads wound around the core thread may have gaps thereamong. Thus, the resulting absorbable suture thread may not have high tensile and knot strengths. A plurality of the winding threads each having a diameter exceeding 0.6 mm may be difficult to wind around the core thread. The respective diameters of the winding threads are more preferably 0.1 mm as the lower limit and 0.5 mm as the upper limit.

Examples of methods for producing the core thread and the winding threads can include, but not particularly limited to, conventionally known spinning methods such as a melt spinning method.

The bioabsorbable suture thread of the present invention has a plurality of the winding threads wound around the core thread and has a structure in which the core thread molten by heat treatment is bonded to the winding threads.

Examples of the form in which a plurality of the winding threads are wound around the core thread include: a twisted yarn form in which a plurality of the winding threads are spirally wound around the core thread; a braided form in which a plurality of the winding threads are wound in a braided manner around the core thread; and a covering thread form in which a plurality of the winding threads are wound in a processed covering manner around the core thread. Among them, a twisted yarn form in which a plurality of the winding threads are spirally wound around the core thread is preferred because a plurality of the winding threads can be continuously wound around the core thread at a high speed and particularly in terms of excellent production efficiency. In such a twisted yarn form, the winding threads are wound around the core thread while twisted. Thus, even in the case of multifilament threads used as the winding threads, all monofilament threads constituting the multifilament threads are inevitably brought into contact with the molten core thread. Thus, this form is significantly effective, particularly, for offering high knot security without untwisting a plurality of threads even at a cut end or a point of torsional bending.

The number of the winding threads wound around the core thread is 3 or more. A bioabsorbable suture thread particularly having high flexibility can thus be obtained. The upper limit of the number of the winding threads wound around the core thread is not particularly limited and is preferably 6 or less in consideration of obtained effects and easy production.

In the bioabsorbable suture thread of the present invention, the core thread molten by heat treatment is bonded to the winding threads. This bonding between the core thread and a plurality of the winding threads is effective for offering excellent knot security without untwisting a plurality of threads even at a cut end or a point of torsional bending.

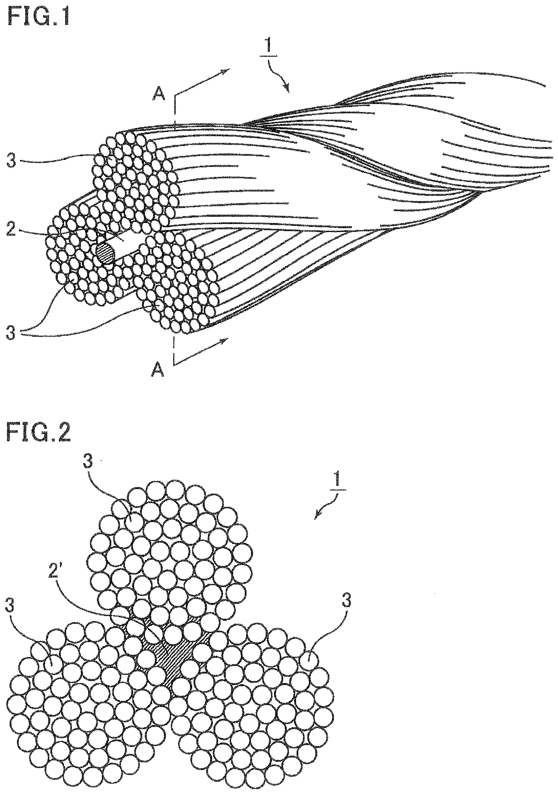

Figure 1 shows a schematic perspective view of one example of the bioabsorbable suture thread of the present invention. A bioabsorbable suture thread 1 shown in Figure 1 employs three winding threads 3 (multifilament threads) as a plurality of the winding threads and is constituted in a twisted yarn form in which these winding threads 3 are spirally wound around a core thread 2.

Figure 2 shows a schematic cross-sectional view taken along the A-A line in the bioabsorbable suture thread 1 of Figure 1. As shown in Figure 2, the core thread 2 is thermally molten by heat treatment and dispersed as a thermally molten mass 2' to form a structure bonded to the multifilament threads 3 (multifilament threads).

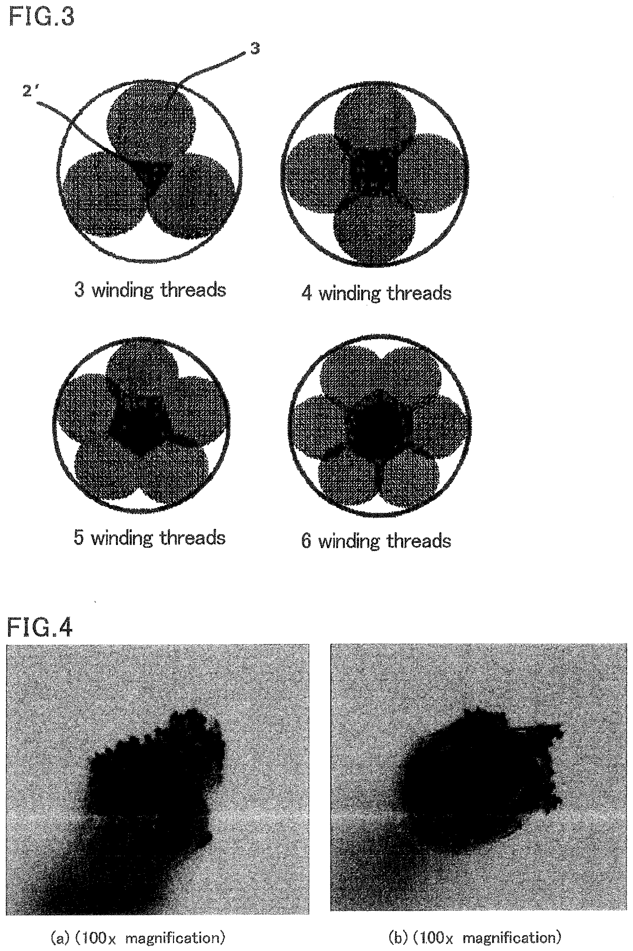

Figure 3 shows a schematic cross-sectional view of the bioabsorbable suture thread of the present invention having 3 to 6 winding threads.

The bioabsorbable suture thread of the present invention may further have, on its surface, a coating layer made of a lubricant or the like. The lubricant is not particularly limited, and a conventionally known lubricant such as silicone or sugar ester may be used. Alternatively, the bioabsorbable suture thread of the present invention may further have a covering layer containing higher fatty acid salt, at least one bioabsorbable polymer selected from the group consisting of polycaprolactone, a caprolactone-lactic acid copolymer, a caprolactone-glycolic acid copolymer, polylactic acid, and a lactic acid-glycolic acid copolymer, sucrose fatty acid ester, or the like, as described in Japanese Patent No. 2958507.

Preferably, the bioabsorbable suture thread of the present invention has a diameter of 0.03 to 0.8 mm. The bioabsorbable suture thread having a diameter smaller than 0.03 mm might not have strengths required for suture threads. The bioabsorbable suture thread having a diameter exceeding 0.8 mm might be unsuitable for use as a suture thread due to its rigidity. The diameter is more preferably 0.035 mm as the lower limit and 0.7 mm as the upper limit.

Preferably, the bioabsorbable suture thread of the present invention has a tensile strength of 0.5 N or higher. The bioabsorbable suture thread having a tensile strength lower than 0.5 N might not have strengths required for suture threads. The tensile strength is more preferably 1 N or higher.

In the present specification, the tensile strength means a value obtained by measurement according to the method of JIS L1013:2010.

Preferably, the bioabsorbable suture thread of the present invention has an elongation percentage of 5% to 70%. The bioabsorbable suture thread having an elongation percentage less than 5% might be difficult to handle. The bioabsorbable suture thread having an elongation percentage exceeding 70% might not be tied in a knot. The elongation percentage is more preferably 10% to 60%.

In the present specification, the elongation percentage means a value obtained by measurement according to the method of JIS L1013:2010.

Preferably, the bioabsorbable suture thread of the present invention has a knot strength of 0.5 N or higher. If the knot strength is lower than 0.5 N, a knot might be broken in use of the suture thread. The knot strength is more preferably 0.6 N or higher.

In the present specification, the knot strength means a value obtained by measurement according to the method prescribed by the United States Pharmacopoeia (USP).

Examples of methods for producing the bioabsorbable suture thread of the present invention include an internal bond-based thread production method which involves winding a plurality of the winding threads around the core thread, followed by heat treatment. The internal bond-based thread production method is specifically described in, for example, Japanese Patent No. 3205556.

The method for producing the bioabsorbable suture thread of the present invention will be described more specifically by taking a twisted yarn form in which a plurality of the winding threads are spirally wound around the core thread, as an example of the form in which a plurality of the winding threads are wound around the core thread.

Specifically, the step of concurrently twisting, in the same direction, each of a plurality of the winding threads to prepare twisted threads is first performed. Subsequently, the step of twisting a plurality of the twisted threads thus obtained in combination with the core thread, in a direction opposite to the twisting direction of a plurality of the winding threads is performed. As a result, the core thread is enclosed in the center of twisted yarn with a plurality of the winding threads to obtain twisted yarn in which a plurality of the winding threads are spirally wound around the core thread.

Subsequently, the core thread is molten by heat treatment to bond the core thread to a plurality of the winding threads. Examples of methods for the heat treatment include, but not particularly limited to: a method which involves reeling, on a heated drum, the obtained twisted yarn in which a plurality of the winding threads are spirally wound around the core thread; a method which involves winding the twisted yarn on a bobbin and leaving the resulting product standing in a constant-temperature zone; and a method which involves moving the twisted yarn at a constant speed in a heat treatment apparatus.

The heat treatment is performed at a temperature that is equal to or higher than the melting point of the bioabsorbable lower-melting component constituting the core thread and is lower than the melting point of the bioabsorbable higher-melting component constituting the winding threads. Specifically, the heat treatment is preferably performed, for example, at 80 to 180°C for 30 to 90 minutes, more preferably at 90 to 150°C for 40 to 80 minutes.

According to one aspect, the present disclosure also provides a method for producing the bioabsorbable suture thread of the present invention, comprising the steps of: concurrently twisting, in the same direction, each of a plurality of winding threads made of a bioabsorbable higher-melting component to prepare twisted threads; twisting a plurality of the twisted threads thus obtained in combination with a core thread made of a bioabsorbable lower-melting component, in a direction opposite to the twisting direction of a plurality of the winding threads; and melting the core thread by heat treatment at a temperature that is equal to or higher than the melting point of the bioabsorbable lower-melting component constituting the core thread and is lower than the melting point of the bioabsorbable higher-melting component constituting the winding threads to bond the core thread to a plurality of the winding threads.

The above production method for the bioabsorbable suture thread can produce a suture thread of approximately 5 to 7 m per minute and achieves the effect of improving productivity by 60 times that of the conventional production method for a braided suture thread using a braiding machine, which can merely produce a suture thread of approximately 5 to 10 m per hour.

- Advantageous Effects of Invention

The bioabsorbable suture thread of the present invention is constituted as mentioned above. The bioabsorbable suture thread of the present invention therefore has high flexibility and high tensile and knot strengths, maintains its figure without untwisting a plurality of threads even at a cut end or a point of torsional bending, and is excellent in knot security and production efficiency. Thus, the bioabsorbable structure thread can be preferably applied as a surgical suture thread.

BRIEF DESCRIPTION OF DRAWINGS

Figure 1 shows a schematic perspective view of one example of the bioabsorbable suture thread of the present invention.

Figure 2 shows a schematic cross-sectional view of one example of the bioabsorbable suture thread of the present invention.

Figure 3 shows a schematic cross-sectional view of the bioabsorbable suture thread of the present invention having 3 to 6 winding threads.

Figure 4 is micrographs of the cut cross-sections of a suture thread obtained in Example 1 (Figure 4(a)) and a suture thread obtained in Comparative Example 2 (Figure 4(b)).

Figure 5 is micrographs of the points of torsional bending of the suture thread obtained in Example 1 (Figure 5(a)) and the suture thread obtained in Comparative Example 2 (Figure 5(b)).

DESCRIPTION OF EMBODIMENTS

Hereinafter, the present invention will be described in more detail with reference to Examples. In Examples below, "%" and "part" mean "% by mass" and "part by mass", respectively, unless otherwise specified.

(Example 1)

A monofilament thread (diameter: 0.16 mm) made of ε-polycaprolactone having a melting point of 65°C was used as a core thread. Three polyglycolic acid multifilament threads each consisting of 184 single polyglycolic acid filaments each having a thread diameter of 0.019 mm were wound around the core thread to produce twisted yarn. This twisted yarn was reeled on a heat treatment drum and continuously heat-treated at 110°C for 60 minutes to obtain a bioabsorbable suture thread corresponding to USP No. 1. The obtained suture thread kept its figure as a multifilament suture thread without untwisting a plurality of the polyglycolic acid multifilament threads wound around the core thread.

(Example 2)

A monofilament thread (diameter: 0.164 mm) made of ε-polycaprolactone having a melting point of 65°C was used as a core thread. Three polyglycolic acid multifilament threads each consisting of 92 single polyglycolic acid filaments each having a thread diameter of 0.019 mm were wound around the core thread to produce twisted yarn. This twisted yarn was reeled on a heat treatment drum and continuously heat-treated at 110°C for 60 minutes to obtain a bioabsorbable suture thread corresponding to USP No. 2-0. The obtained suture thread kept its figure as a multifilament suture thread without untwisting a plurality of the polyglycolic acid multifilament threads wound around the core thread.

(Example 3)

A monofilament thread (diameter: 0.160 mm) made of ε-polycaprolactone having a melting point of 65°C was used as a core thread. Three polyglycolic acid multifilament threads each consisting of 69 single polyglycolic acid filaments each having a thread diameter of 0.018 mm were wound around the core thread to produce twisted yarn. This twisted yarn was reeled on a heat treatment drum and continuously heat-treated at 110°C for 60 minutes to obtain a bioabsorbable suture thread corresponding to USP No. 3-0. The obtained suture thread kept its figure as a multifilament suture thread without untwisting a plurality of the polyglycolic acid multifilament threads wound around the core thread.

(Example 4)

A monofilament thread (diameter: 0.080 mm) made of ε-polycaprolactone having a melting point of 65°C was used as a core thread. Three polyglycolic acid multifilament threads each consisting of 46 single polyglycolic acid filaments each having a thread diameter of 0.018 mm were wound around the core thread to produce twisted yarn. This twisted yarn was reeled on a heat treatment drum and continuously heat-treated at 110°C for 60 minutes to obtain a bioabsorbable suture thread corresponding to USP No. 4-0. The obtained suture thread kept its figure as a multifilament suture thread without untwisting a plurality of the polyglycolic acid multifilament threads wound around the core thread.

(Example 5)

A monofilament thread (diameter: 0.080 mm) made of a glycolic acid-ε-caprolactone copolymer (ε-polycaprolactone:glycolic acid copolymerization ratio = 50:50%) having a melting point of 90°C was used as a core thread. Three polyglycolic acid multifilament threads each consisting of 46 single polyglycolic acid filaments each having a thread diameter of 0.018 mm were wound around the core thread to produce twisted yarn. This twisted yarn was reeled on a heat treatment drum and continuously heat-treated at 180°C for 60 minutes to obtain a bioabsorbable suture thread corresponding to USP No. 4-0. The obtained suture thread kept its figure as a multifilament suture thread without untwisting a plurality of the polyglycolic acid multifilament threads wound around the core thread.

(Example 6)

A monofilament thread (diameter: 0.080 mm) made of a lactic acid-ε-caprolactone copolymer (ε-polycaprolactone:lactic acid copolymerization ratio = 50:50%) having a melting point of 85°C was used as a core thread. Three polyglycolic acid multifilament threads each consisting of 46 single polyglycolic acid filaments each having a thread diameter of 0.018 mm were wound around the core thread to produce twisted yarn. This twisted yarn was reeled on a heat treatment drum and continuously heat-treated at 160°C for 60 minutes to obtain a bioabsorbable suture thread corresponding to USP No. 4-0. The obtained suture thread kept its figure as a multifilament suture thread without untwisting a plurality of the polyglycolic acid multifilament threads wound around the core thread.

(Example 7)

A monofilament thread (diameter: 0.164 mm) made of ε-polycaprolactone having a melting point of 65°C was used as a core thread. Three polyglycolic acid-lactic acid copolymer multifilament threads each consisting of 92 single glycolic acid-lactic acid copolymer (glycolic acid:lactic acid copolymerization ratio = 90:10%) filaments each having a thread diameter of 0.019 mm were wound around the core thread to produce twisted yarn. This twisted yarn was reeled on a heat treatment drum and continuously heat-treated at 110°C for 60 minutes to obtain a bioabsorbable suture thread corresponding to USP No. 2-0. The obtained suture thread kept its figure as a multifilament suture thread without untwisting a plurality of the polyglycolic acid-lactic acid copolymer multifilament threads wound around the core thread.

(Example 8)

A monofilament thread (diameter: 0.164 mm) made of s-polycaprolactone having a melting point of 65°C was used as a core thread. Three polyglycolic acid-lactic acid copolymer multifilament threads each consisting of 46 single glycolic acid-lactic acid copolymer (glycolic acid:lactic acid copolymerization ratio = 90:10%) filaments each having a thread diameter of 0.019 mm were wound around the core thread to produce twisted yarn. This twisted yarn was reeled on a heat treatment drum and continuously heat-treated at 110°C for 60 minutes to obtain a bioabsorbable suture thread corresponding to USP No. 4-0. The obtained suture thread kept its figure as a multifilament suture thread without untwisting a plurality of the polyglycolic acid-lactic acid copolymer multifilament threads wound around the core thread.

(Example 9)

A ε-polycaprolactone multifilament thread consisting of 50 single filaments each made of ε-polycaprolactone having a melting point of 65°C and a thread diameter of 0.003 mm was used as a core thread. Six polyglycolic acid-lactic acid copolymer multifilament threads each consisting of 144 single glycolic acid-lactic acid copolymer (glycolic acid:lactic acid copolymerization ratio = 90:10%) filaments each having a thread diameter of 0.019 mm were wound around the core thread to produce twisted yarn. This twisted yarn was reeled on a heat treatment drum and continuously heat-treated at 110°C for 60 minutes to obtain a bioabsorbable suture thread corresponding to USP No. 2. The obtained suture thread kept its figure as a multifilament suture thread without untwisting a plurality of the polyglycolic acid multifilament threads wound around the core thread.

(Example 10)

A s-polycaprolactone multifilament thread consisting of 40 single filaments each made of ε-polycaprolactone having a melting point of 65°C and a thread diameter of 0.003 mm was used as a core thread. Five polyglycolic acid-lactic acid copolymer multifilament threads each consisting of 144 single glycolic acid-lactic acid copolymer (glycolic acid:lactic acid copolymerization ratio = 90:10%) filaments each having a thread diameter of 0.019 mm were wound around the core thread to produce twisted yarn. This twisted yarn was reeled on a heat treatment drum and continuously heat-treated at 110°C for 60 minutes to obtain a bioabsorbable suture thread corresponding to USP No. 1. The obtained suture thread kept its figure as a multifilament suture thread without untwisting a plurality of the polyglycolic acid multifilament threads wound around the core thread.

(Example 11)

A ε-polycaprolactone multifilament thread consisting of 30 single filaments each made of ε-polycaprolactone having a melting point of 65°C and a thread diameter of 0.003 mm was used as a core thread. Four glycolic acid-lactic acid copolymer multifilament threads each consisting of 114 single glycolic acid-lactic acid copolymer (glycolic acid:lactic acid copolymerization ratio = 90:10%) filaments each having a thread diameter of 0.019 mm were wound around the core thread to produce twisted yarn. This twisted yarn was reeled on a heat treatment drum and continuously heat-treated at 110°C for 60 minutes to obtain a bioabsorbable suture thread corresponding to USP No. 1-0. The obtained suture thread kept its figure as a multifilament suture thread without untwisting a plurality of the polyglycolic acid multifilament threads wound around the core thread.

(Example 12)

A ε-polycaprolactone multifilament thread consisting of 20 single filaments each made of ε-polycaprolactone having a melting point of 65°C and a thread diameter of 0.008 mm was used as a core thread. Three glycolic acid-lactic acid copolymer multifilament threads each consisting of 114 single glycolic acid-lactic acid copolymer (glycolic acid:lactic acid copolymerization ratio = 90:10%) filaments each having a thread diameter of 0.019 mm were wound around the core thread to produce twisted yarn. This twisted yarn was reeled on a heat treatment drum and continuously heat-treated at 110°C for 60 minutes to obtain a bioabsorbable suture thread corresponding to USP No. 2-0. The obtained suture thread kept its figure as a multifilament suture thread without untwisting a plurality of the polyglycolic acid multifilament threads wound around the core thread.

(Example 13)

A ε-polycaprolactone multifilament thread consisting of 10 single filaments each made of ε-polycaprolactone having a melting point of 65°C and a thread diameter of 0.02 mm was used as a core thread. Three glycolic acid-lactic acid copolymer multifilament threads each consisting of 63 single glycolic acid-lactic acid copolymer (glycolic acid:lactic acid copolymerization ratio = 90:10%) filaments each having a thread diameter of 0.019 mm were wound around the core thread to produce twisted yarn. This twisted yarn was reeled on a heat treatment drum and continuously heat-treated at 110°C for 60 minutes to obtain a bioabsorbable suture thread corresponding to USP No. 3-0. The obtained suture thread kept its figure as a multifilament suture thread without untwisting a plurality of the polyglycolic acid multifilament threads wound around the core thread.

(Example 14)

A ε-polycaprolactone multifilament thread consisting of 8 single filaments each made of ε-polycaprolactone having a melting point of 65°C and a thread diameter of 0.01 mm was used as a core thread. Three glycolic acid-lactic acid copolymer multifilament threads each consisting of 42 single glycolic acid-lactic acid copolymer (glycolic acid:lactic acid copolymerization ratio = 90:10%) filaments each having a thread diameter of 0.019 mm were wound around the core thread to produce twisted yarn. This twisted yarn was reeled on a heat treatment drum and continuously heat-treated at 110°C for 60 minutes to obtain a bioabsorbable suture thread corresponding to USP No. 4-0. The obtained suture thread kept its figure as a multifilament suture thread without untwisting a plurality of the polyglycolic acid multifilament threads wound around the core thread.

(Comparative Example 1)

A suture thread consisting of 3 polyglycolic acid multifilament threads was prepared without use of the core thread in Example 1. The obtained suture thread became immediately untwisted from the end of the thread and was thus unable to keep its figure as a multifilament suture thread.

(Comparative Examples 2 to 5)

Commercially available USP Nos. 1, 2-0, 3-0, and 4-0 braided suture threads (trade name: VICRYL, manufactured by Ethicon, Inc.) consisting of glycolic acid-lactic acid copolymer multifilament threads were used.

(Evaluation)

The suture threads obtained in Examples and Comparative Examples were evaluated for items shown below. However, the suture thread of Comparative Example 1 was unable to keep its figure and thus, was not evaluated.
The results are shown in Table 1.

(1) Evaluation of tensile strength

The respective tensile strengths of the suture threads obtained in Examples and Comparative Examples were measured by a method according to JIS L1013:2010. Specifically, the test was conducted at a speed of 100 mm/min. using a tensile tester (Tensile Tester Model 4302 manufactured by Instron Japan Co., Ltd.) with a chuck distance set to 100 mm. The strength of each suture thread when the thread was cut was measured as the tensile strength.

(2) Evaluation of knot strength

The respective knot strengths of the suture threads obtained in Examples and Comparative Examples were measured according to the method prescribed by the United States Pharmacopoeia (USP). Each suture thread was tied in a surgical knot with its central portion wound on a flexible rubber tube having an outside diameter of 100 mm. The thread was fixed at both ends to chucks with a chuck distance set to 100 mm so that the knot was positioned at the center. The test was conducted at a speed of 100 mm/min. using a tensile tester (Tensile Tester Model 4302 manufactured by Instron Japan Co., Ltd.). The load of the suture thread when the thread was cut was defined as the knot strength.

(3) Evaluation of knot security

Each of the suture threads obtained in Examples and Comparative Examples was firmly tied in a square knot around a stainless bar having a diameter of 100 mm. A load of 30 g was applied to the loop portion. A plastic bar was moved 2000 times at a speed of 100 times/min. on the knot of the suture thread. The suture thread was visually observed and evaluated as "○" when its knot did not come loose and as "×" when looseness was observed in its knot.

(4) Evaluation of flexibility

The suture threads obtained in Examples 9 to 14 and Comparative Examples 2 to 5 were each evaluated for flexibility according to the method described in "G.T. Rodeheaver et al., J. Long-Term Effect of Medical Implants, Vol. 6, p. 181-198 (1996)".

Specifically, each obtained suture thread was cut into a length of 20 cm and weighted at both ends. The center of the thread was hung on a hook having a diameter of 0.9 mm in an environment involving a temperature of 20°C and a humidity of 50%. The distance between the ends of the thread was measured with the weights spontaneously hanging. The degree G of flexibility of the thread was determined according to the expression shown below. The measurement was performed five times per sample, and an average value thereof was determined.

$$G = WL^3 / 3d$$

wherein W represents the weight of the weights (in this method, 0.00638 N); L represents the length (in this method, 20 cm) of the suture thread used in the test; and d represents the measured distance (cm) between the ends of the suture thread with the weights hanging.

(5) Evaluation of figure-holding performance (cut surface)

Each of the suture threads obtained in Examples and Comparative Examples was cut using surgical scissors. The cut surface was visually observed using a microscope at a magnification of ×100 and evaluated as "○" when the threads were not untwisted and as "×" when the threads were partially untwisted.

Figure 4(a) shows a micrograph of the cut cross-section of the suture thread obtained in Example 1. Figure 4(b) shows a micrograph of the cut cross-section of the suture thread obtained in Comparative Example 2.

(6) Evaluation of figure-holding performance (point of torsional bending)

Each of the suture threads obtained in Examples and Comparative Examples was bended 90° or larger with torsion applied thereto to prepare a point of torsional bending. The point of torsional bending was visually observed using a microscope at a magnification of ×200 and evaluated as "○" when the threads were not untwisted and as "×" when the threads were partially untwisted.

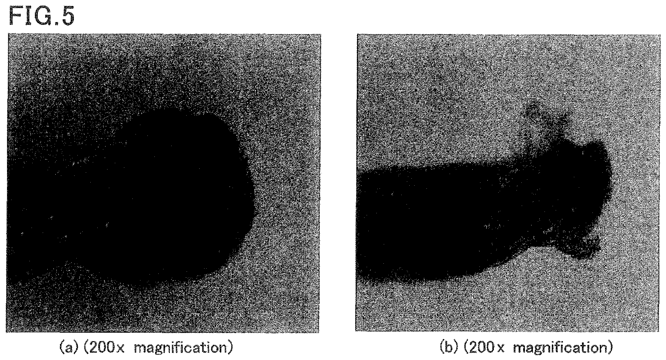

Figure 5(a) shows a micrograph of the point of torsional bending of the suture thread obtained in Example 1. Figure 5(b) shows a micrograph of the point of torsional bending of the suture thread obtained in Comparative Example 2.

[Table 1]

| | USP No. | Suture thread | | | | | | | Evaluation | | | | | |
| | | Core thread | | | Winding thread | | | | Tensile strength (N) | Knot strength (N) | Knot security | Flexibility (G value) | Figure-holding per-formance | |
| | | Material | Single thread di-ameter (mm) | Number of threads | Material | Single thread diameter (mm) | Number of threads | Number of winding threads | | | | | Cut surface | Point of torsional bending |
|---|---|---|---|---|---|---|---|---|---|---|---|---|---|---|
| Example 1 | 1 | PCL | 0.16 | 1 | PGA | 0.019 | 184 | 3 | 113.0 | 65.0 | ○ | - | ○ | ○ |
| Example 2 | 2-0 | PCL | 0.164 | 1 | PGA | 0.019 | 92 | 3 | 57.3 | 36.5 | ○ | - | ○ | ○ |
| Example 3 | 3-0 | PCL | 0.16 | 1 | PGA | 0.018 | 69 | 3 | 41.3 | 26.4 | ○ | - | ○ | ○ |
| Example 4 | 4-0 | PCL | 0.08 | 1 | PGA | 0.018 | 46 | 3 | 25.7 | 17.2 | ○ | - | ○ | ○ |
| Example 5 | 4-0 | P(GA/CL) | 0.08 | 1 | PGA | 0.018 | 46 | 3 | 56.8 | 36.2 | ○ | - | ○ | ○ |
| Example 6 | 4-0 | P(LA/CL) | 0.08 | 1 | PGA | 0.018 | 46 | 3 | 55.4 | 35.3 | ○ | - | ○ | ○ |
| Example 7 | 2-0 | PCL | 0.164 | 1 | PGLA | 0.019 | 92 | 3 | 31.4 | 21.5 | ○ | - | ○ | ○ |
| Example 8 | 4-0 | PCL | 0.164 | 1 | PGLA | 0.019 | 46 | 3 | 23.5 | 16.3 | ○ | - | ○ | ○ |
| Example 9 | 2 | PCL | 0.003 | 50 | PGLA | 0.019 | 144 | 6 | 125.1 | 74.0 | ○ | 22.2 | ○ | ○ |
| Example 10 | 1 | PCL | 0.003 | 40 | PGLA | 0.019 | 144 | 5 | 105.1 | 67.3 | ○ | 18.6 | ○ | ○ |
| Example 11 | 1-0 | PCL | 0.003 | 30 | PGLA | 0.019 | 114 | 4 | 75.6 | 42.4 | ○ | 12.2 | ○ | ○ |
| Example 12 | 2-0 | PCL | 0.008 | 20 | PGLA | 0.019 | 114 | 3 | 54 | 36.2 | ○ | 7.9 | ○ | ○ |
| Example 13 | 3-0 | PCL | 0.02 | 10 | PGLA | 0.019 | 63 | 3 | 32 | 20.3 | ○ | 4.4 | ○ | ○ |
| Example 14 | 4-0 | PCL | 0.01 | 8 | PGLA | 0.019 | 42 | 3 | 20 | 13.6 | ○ | 3.42 | ○ | ○ |
| Comparative Example 1 | 1 | - | - | - | PGA | 0.019 | 184 | 3 | - | - | - | - | - | - |
| Comparative Example 2 | 1 | Commercially available braided suture thread | | | | | | | 132.1 | 60.1 | × | 12.5 | × | × |
| Comparative Example 3 | 2-0 | Commercially available braided suture thread | | | | | | | 72.5 | 36.6 | × | 7.5 | × | × |
| Comparative Example 4 | 3-0 | Commercially available braided suture thread | | | | | | | 43.7 | 21.9 | × | 5.0 | × | × |

(continued)

| | | Suture thread | | | | | | | Evaluation | | | | | Figure-holding performance | |
|---|---|---|---|---|---|---|---|---|---|---|---|---|---|---|---|
| | | Core thread | | | Winding thread | | | | | | | | | | |
| | USP No. | Material | Single thread diameter (mm) | Number of threads | Material | Single thread diameter (mm) | Number of threads | Number of winding threads | Tensile strength (N) | Knot strength (N) | Knot security | Flexibility (G value) | | Cut surface | Point of torsional bending |
| Comparative Example 5 | 4-0 | Commercially available braided suture thread | | | | | | | 31.2 | 15.8 | × | 3.4 | | × | × |

PGA Polyglycolic acid
PGLA Copolymer of glycolic acid and lactic acid
PCL Polycaprolactone
P(GA/CL) Copolymer of glycolic acid and ε -caprolactone
P(LA/CL) Copolymer of lactic acid and ε -caprolactone

11

INDUSTRIAL APPLICABILITY

The bioabsorbable suture thread of the present invention has high flexibility and high tensile and knot strengths, maintains its figure without untwisting a plurality of threads even at a cut end or a point of torsional bending, and is excellent in knot security and production efficiency. Thus, the bioabsorbable suture thread of the present invention can be preferably applied as a surgical suture thread.

REFERENCE SIGNS LIST

1:    Bioabsorbable suture thread
2:    Core thread
2':   Thermally molten mass
3:    Winding threads

## Claims

1. A bioabsorbable suture thread comprising: a core thread made of a bioabsorbable lower-melting component; and a plurality of winding threads made of a bioabsorbable higher-melting component, the winding threads being wound around the core thread,
   wherein the bioabsorbable suture thread has a structure in which the core thread molten by heat treatment is bonded to the winding threads
   wherein the number of the winding threads wound around the core thread is 3 or more,
   wherein the bioabsorbable lower-melting component constituting the core thread has a melting point of 50 to 200°C, and the bioabsorbable higher-melting component constituting the winding threads has a melting point of 180 to 290°C,
   wherein the bioabsorbable lower-melting component constituting the core thread is at least one selected from the group consisting of ε-polycaprolactone, a copolymer of ε-polycaprolactone and glycolic acid, and a copolymer of ε-polycaprolactone and lactic acid,
   wherein the bioabsorbable higher-melting component constituting the winding threads is polyglycolic acid and/or a copolymer of glycolic acid and lactic acid.

2. The bioabsorbable suture thread according to claim 1,
   wherein the bioabsorbable suture thread has a shape of twisted yarn in which a plurality of the winding threads are spirally wound around the core thread.

3. The bioabsorbable suture thread according to claim 1 and 2,
   wherein the winding threads are multifilament threads.

## Patentansprüche

1. Bioabsorbierbarer Nahtfaden, umfassend: einen Kernfaden, der aus einer bioabsorbierbaren niedriger-schmelzenden Komponente hergestellt ist; und eine Vielzahl an Umwickelungsfäden, die aus einer bioabsorbierbaren höher-schmelzenden Komponente hergestellt sind, wobei die Umwickelungsfäden um den Kernfaden herum gewickelt sind, wobei der bioabsorbierbare Nahtfaden eine Struktur hat, in der der durch Wärmebehandlung geschmolzene Kernfaden an die Umwickelungsfäden gebunden ist,
   wobei die Anzahl der Umwickelungsfäden, die um den Kernfaden herumgewickelt sind, 3 oder mehr ist,
   wobei die bioabsorbierbare niedriger-schmelzende Komponente, die den Kernfaden bildet, einen Schmelzpunkt von 50 bis 200°C hat und die bioabsorbierbare höher-schmelzende Komponente, die die Umwickelungsfäden bildet, einen Schmelzpunkt von 180 bis 290°C hat,
   wobei die bioabsorbierbare niedriger-schmelzende Komponente, die den Kernfaden bildet, wenigstens eine, ausgewählt aus der Gruppe, bestehend aus ε-Polycaprolacton, einem Copolymer von ε-Polycaprolacton und Glycolsäure und einem Copolymer von ε-Polycaprolacton und Milchsäure, ist,
   wobei die bioabsorbierbare höher-schmelzende Komponente, die die Umwickelungsfäden bildet, Polyglycolsäure und/oder ein Copolymer von Glycolsäure und Milchsäure ist.

**2.** Bioabsorbierbarer Nahtfaden gemäß Anspruch 1,
wobei der bioabsorbierbare Nahtfaden eine Form von verzwirntem Garn, in der eine Vielzahl der Umwickelungsfäden spiralförmig um den Kernfaden herum gewickelt sind, hat.

**3.** Bioabsorbierbarer Nahtfaden gemäß Anspruch 1 und 2,
wobei die Umwickelungsfäden Multifilamentfäden sind.

**Revendications**

**1.** Fil de suture biorésorbable comprenant : un fil central constitué d'un composant biorésorbable à point de fusion plus bas ; et une pluralité de fils d'enroulement constitués d'un composant biorésorbable à point de fusion plus élevé, les fils d'enroulement étant enroulés autour du fil central,
dans lequel le fil de suture biorésorbable a une structure dans laquelle le fil central fondu par traitement thermique est lié aux fils d'enroulement,
dans lequel le nombre des fils d'enroulement enroulés autour du fil central est de 3 ou plus,
dans lequel le composant biorésorbable à point de fusion plus bas constituant le fil central a un point de fusion de 50 à 200 °C et le composant biorésorbable à point de fusion plus élevé constituant les fils d'enroulement a un point de fusion de 180 à 290 °C,
dans lequel le composant biorésorbable à point de fusion plus bas constituant le fil central est au moins un fil choisi dans le groupe constitué de la ε-polycaprolactone, d'un copolymère de ε-polycaprolactone et d'acide glycolique et d'un copolymère de ε-polycaprolactone et d'acide lactique,
dans lequel le composant biorésorbable à point de fusion plus élevé constituant les fils d'enroulement est l'acide polyglycolique et/ou un copolymère d'acide glycolique et d'acide lactique.

**2.** Fil de suture biorésorbable selon la revendication 1,
dans lequel le fil de suture biorésorbable a une forme de fil retordu dans laquelle une pluralité des fils d'enroulement est enroulée en spirale autour du fil central.

**3.** Fil de suture biorésorbable selon les revendications 1 et 2,
dans lequel les fils d'enroulement sont des fils multifilamentaires.

EP 2 687 238 B1

FIG.1

FIG.2

14

FIG.3

3 winding threads      4 winding threads

5 winding threads      6 winding threads

FIG.4

(a) (100x magnification)      (b) (100x magnification)

15

FIG.5

(a) (200× magnification)

(b) (200× magnification)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI0614987 B
- JP H0614987 A
- JP 2008113790 A
- JP 2004250853 A
- US 4470941 A
- WO 03092758 A1
- WO 9303213 A1
- JP 2958507 B
- JP 3205556 B

**Non-patent literature cited in the description**

- **G.T. RODEHEAVER et al.** *J. Long-Term Effect of Medical Implants,* 1996, vol. 6, 181-198